(19) Europäisches Patentamt
European Patent Office
Office européen des brevets

(11) Veröffentlichungsnummer: **0 127 114**
**B1**

(12) # EUROPÄISCHE PATENTSCHRIFT

(45) Veröffentlichungstag der Patentschrift: **29.10.86**

(21) Anmeldenummer: **84105830.8**

(22) Anmeldetag: **22.05.84**

(51) Int. Cl.[4]: **C 07 C 85/20**, C 07 C 93/14, C 07 C 121/66, C 07 C 121/78, C 07 C 103/28, C 07 C 95/08, C 07 C 91/16, C 07 C 147/12, C 07 D 233/95, C 07 C 149/42, C 07 D 295/12

(54) **Verfahren zur Spaltung von Phthalimiden.**

(30) Priorität: **31.05.83 DE 3319650**

(43) Veröffentlichungstag der Anmeldung: **05.12.84 Patentblatt 84/49**

(45) Bekanntmachung des Hinweises auf die Patenterteilung: **29.10.86 Patentblatt 86/44**

(84) Benannte Vertragsstaaten: **CH DE FR GB LI**

(56) Entgegenhaltungen:
**DE - A - 2 749 415**
**US - A - 4 359 584**

**CHEMICAL ABSTRACTS, Band 56, Nr. 1, 8. Jänner 1962, Columbus, Ohio, USA KAZUO NAKAJIMA "Condensation reactions of amino alcohols with imides. I. Condensation productus on N,N-diethylamino-ethanol with various imides; formation of N,N-diethyl-ethylene diamine" Spalte 404, Zusammenfassung-Nr. 405a**
**CHEMICAL ABSTRACTS, Band 50, Nr. 20, 25. Oktober 1956, Columbus, Ohio, USA N. RABJOHN et al. "Some reactions of N-acetylphtalimides" Spalte 14 644, Zusammenfassung-Nr. 14 645b**
**CHEMICAL ABSTRACTS, Band 60, Nr. 5, 2. März 1964, Columbus, Ohio, USA R. BORISAVLJEVIC et al. "N-Benzoylphtalimide. II. Reaction with amines" Spalte 5 390, Zusammenfassung-Nr. 5 390g**

(73) Patentinhaber: **BASF Aktiengesellschaft, Carl-Bosch-Strasse 38, D-6700 Ludwigshafen (DE)**

(72) Erfinder: **Hagen, Helmut, Dr., Max-Slevogt-Strasse 17e, D-6710 Frankenthal (DE)**
Erfinder: **Kohler, Rolf-Dieter, Dr., Amselweg 3, D-6803 Edingen-Neckarhausen (DE)**

(56) Entgegenhaltungen: (Fortsetzung)
**NATURE, Band 158, 1946, London H.J. BARKER et al. "Compositions of the antimalarial drug R. 63 and the Ing and Manske hydrazine hydrolysis of N-substituted phtalimides" Seite 514**
**CHEMICAL ABSTRACTS, Band 74, Nr. 7, 15. Februar 1971, Columbus, Ohio, USA S. WOLFE et al. "Five-membered rings. II. Inter- and intramolecular reactions of simple amines with N-substituted phtalimides. Methylamine as a reagent for removal of a phthaloyl group from nitrogen" Seite 323, Zusammenfassung-Nr. 31 956y**
**CHEMICAL ABSTRACTS, Band 90, Nr. 11, 12. März 1979, Columbus, Ohio, USA R. ALLAN et al. "Synthesis of some substituted 4-aminobut-2-enoic acids as analogs of the neurotransmitter GABA (alpha-amino-butyric acid)" Seite 665, Zusammenfassung-Nr. 87 858r**

**EP 0 127 114 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Spaltung von Phthalimiden der Formel I

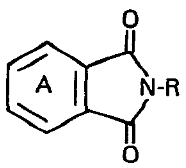

in der

R gegebenenfalls substituiertes Aryl oder Hetaryl ist, das dadurch gekennzeichnet ist, dass man die Verbindungen der Formel I mit Alkanolaminen behandelt.

Als Rest R kommen z.B. gegebenenfalls duch Halogen, Nitro, Alkyl, Alkoxy, Cyan, Carbamoyl, Formyl, Sulfamoyl, Cyanmethyl, Alkoximethyl, Aryloxymethyl, Arylthiomethyl, Arylsulfonylmethyl, Aryl, Benzoyl, Vinylaryl, Vinylimidazolyl oder Carboxyl substituiertes Aryl oder Hetaryl in Betracht.

Einzelne Reste R sind z.B. $C_6H_4Cl$, $C_6H_4NO_2$, $C_6H_4CH_3$, $C_6H_4OCH_3$, $C_6H_3Cl_2$, $C_6H_3(NO_2)_2$, $C_6H_4$-CN, $C_6H_4COOH$, $C_6H_4CONH_2$, $C_6H_4CHO$, $C_6H_4SO_2$-$C_6H_5$, $C_6H_4CH_2CN$, $C_6H_4OCH_3$, $C_6H_4CH_2OCH_3$, $C_6H_4CH_2SCH_3$, $C_6H_4CH_2SC_6H_5$, $C_6H_4CH_2SO_2C_6H_5$, $C_6H_5$ oder $C_6H_4COC_6H_5$.

Die Verbindungen der Formel I können nach an sich bekannten Methoden hergestellt werden, beispielsweise durch Umsetzung einer Halogenverbindung mit Phthalimidkalium nach Gabriel.

Die Spaltung der Phthalimide zu den Aminen und Phthalsäuren oder Derivaten davon stösst in der Praxis häufig auf grosse Schwierigkeiten. Die saure Hydrolyse mit 20 bis 30%iger Salzsäure erfordert meist langes Erhitzen unter Rückfluss [Liebig Ann. Chem. 1949, 22] oder Temperaturen von 200°C unter Druck [Chem. Ber. 20, 2224 (1887)]. Die alkalische Hydrolyse mit wässrigen Laugen bleibt im allgemeinen auf der Stufe der Phthaliminsäuren stehen. Zur vollständigen Hydrolyse muss eine Behandlung mit Mineralsäuren nachgeschaltet werden [Chem. Ber. 37, 1938 (1904)]. Die Spaltung der Phthalimide der Formel I mittels Hydrazin bereitet im Labormassstab zwar kaum Probleme, in technischen Dimensionen erwachsen jedoch grosse Schwierigkeiten. Das bei der Reaktion entstehende schwerlösliche Salz des cyclischen Phthalsäurehydrazids [Nature (London) 158, 514 (1946)] fällt als voluminöser Niederschlag aus, so dass zur Handhabung grosse Mengen an Lösungsmitteln und grosse Reaktionskessel benötigt werden. Die akute Toxizität und der hohe Preis von Hydrazin stehen einer wirtschaftlichen Anwendung ebenfalls hinderlich im Weg.

Es zwar deshalb ausserordentlich überraschend, dass es erfindungsgemäss leicht gelingt, Phthalimide zu spalten.

Die Spaltung der Phthalimide der Formel I geschieht durch einfaches Erwärmen der Verbindungen in Alkanolaminen. Diese fungieren sowohl als Lösungsmittel wie auch als Reaktionskomponenten. Als Alkanolamine geeignet sind z.B. Monoethanolamin, Mono-isopropanolamin, 3-Aminopropanol oder Amino-ethylethanolamin. Vorzugsweise findet Monoethanolamin Verwendung. Die Reaktionstemperaturen liegen in einem Bereich von 40 bis 140°C, vorzugsweise bei 60 bis 100°C. Die Aufarbeitung der Reaktionsmischung hängt von de Natur des freigesetzten Amins ab und ist in der Regel sehr einfach. Beispielsweise kann man wasserunlösliche, feste Amine normalerweise durch Zugabe von Wasser abscheiden. Wasserlösliche Amine können durch Extraktion mit einem Lösungsmittel wie Methylenchlorid, Essigester oder Toluol isoliert werden. Die bei den Umsetzungen freigesetzten Alkanolamide der Phthalsäure verbleiben in der wässrigen Phase und können deshalb problemlos abgetrennt werden.

Man kann natürlich die Spaltung auch in Gegenwart zusätzlicher Lösungsmittel vornehmen, insbesondere, wenn die Spaltprodukte mit den Alkanolaminen reagieren können und somit ein Überschuss Alkanolamin über die stöchiometrisch erforderliche Menge hinaus unzweckmässig ist. Zum Beispiel empfiehlt es sich in Gegenwart austauschbarer Halogenatome nur mit ungefähr stöchiometrischen Mengen an Alkanolaminen zu arbeiten.

Bei der Spaltung verwendbare Lösungsmittel sind beispielsweise Alkanole, Glykole, Glykolether, Ketone, Halogenkohlenwasserstoffe oder Kohlenwasserstoffe. Im einzelnen seien z.B. Methanol, Ethanol, Propanol, Butanol, Glykol, Methyl- oder Ethylglykol, Aceton, Methylethylketon, Tetrahydrofuran, Dioxan, Methylenchlorid, Chlorbenzol oder Toluol genannt.

Der Phthalimidrest dient in der Regel als Schutzgruppe, d.h. seine Anwesenheit ermöglicht Reaktionen, die in Gegenwart einer feien Aminogruppe nicht möglich sind. Beispielsweise können bei geschützter Aminogruppe Alkylierungen, Acylierungen, Nitrierung, Halogenierungen, Chlorsulfonierungen oder Oxidationen vorgenommen werden.

Besonders wertvoll ist das neue Verfahren für Verbindungen mit arylischen oder heteroarylischen Resten R, insbesondere Phenyl-, Naphthyl-, Thiazolyl-, Thiadiazolyl-, Imidazolyl-, Benzthiazolyl-, Benzimidazolyl-, Pyridyl- ode Chinolinresten.

*Beispiel 1*

*2-Amino-benzylcyanid*

100 g 2-Cyanomethyl-N-phenyl-phthalimid wurden bei 80°C portionsweise in 100 g Monoethanolamin eingebracht. Nach 10 Minuten wurde auf 20°C gekühlt und 400 g Eiswasser wurden zugetropft. Das ausgefallene Produkt wurde abgesaugt und mit Eiswasser neutral gewaschen.

Ausbeute: 40 g (80% d.Th.) Fp. 70°C.

*Beispiel 2*

*1-(2-Aminobenzyl)-imidazol*

30 g 2-(1-Imidazolylmethyl)-N-phenyl-phthalimid wurden in 60 g Monoethanolamin 1 Stunde bei 70°C gerührt. Nach dem Abkühlen auf 20°C wurden 200 g Eiswasser zugetropft und die Mischung dreimal mit je 150 g Essigester extrahiert. Die vereinigten Extrakte wurden getrocknet und im Vakuum eingeengt.

Ausbeute: 13 g (75% d.Th.) Fp. 42°C.

*Beispiel 3*

*4,4'-Diamino-benzophenon*

20 g Bis-[4,4'-(Carboxi-phthalimidoyl)]-benzophenon wurden in 30 g Monoethanolamin 15 Minuten bei 80°C gerührt. Anschliessend wurden bei 20°C 200 g Eiswasser zugetropft. Der Niederschlag wurde abgesaugt, mit Wasser gewaschen und getrocknet.

Ausbeute: 7 g (92% d.Th.) Fp. 236°C.

Entsprechend den Beispielen 1 bis 3 wurden die folgenden Amine aus den Verbindungen der Formel I hergestellt:

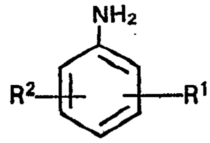

| Beispiel | $R^1$ | $R^2$ | Fp. [°C] |
|---|---|---|---|
| 4 | 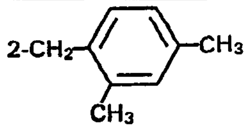 | H | 38 |
| 5 | 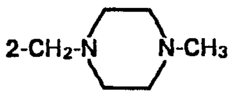 | H | 86 |
| 6 | 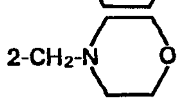 | H | 64 |
| 7 | 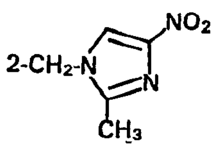 | H | 150 |
| 8 | 2-CN | H | 48 |
| 9 | 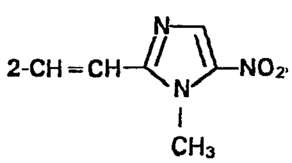 | H | 223 |
| 10 | $2\text{-}CH_2OCH_3$ | H | Öl |
| 11 | 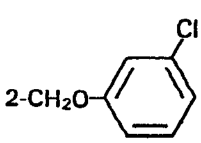 | H | 90 |
| 12 | 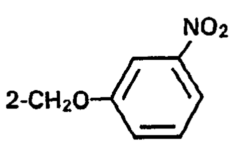 | H | 106 |
| 13 | $2\text{-}CH_2SH$ | H | Öl |
| 14 | $2\text{-}CH_2S\text{-}C_6H_5$ | H | 75 |

| Beispiel | $R^1$ | $R^2$ | Fp. [°C] |
|---|---|---|---|
| 15 | $2\text{-}CH_2SO_2\text{-}C_6H_5$ | H | 178 |
| 16 | 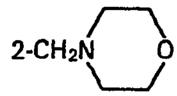 | 3-Cl | 76 |
| 17 | $2\text{-}CH_2OCH_3$ | 3-Cl | Öl |
| 18 | 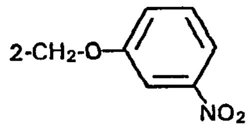 | 3-Cl | 97 |
| 19 | 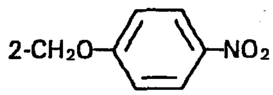 | 3-Cl | 158 |
| 20 | $2\text{-}CH_2SO_2C_6H_5$ | $6\text{-}CH_2SO_2C_6H_5$ | 216 |
| 21 | 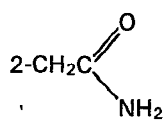 | $4\text{-}NO_2$ | 227 |
| 22 | 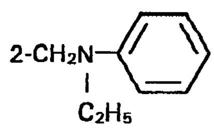 | H | 72 |
| 23 | 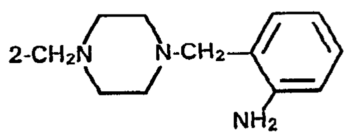 | H | 201 |
| 24 | 2-CH = CH-CN | $-4NO_2$ | 266 |
| 25 | 2-CHO | $4\text{-}NO_2$ | 200 |
| 26 | 2-CHO | 4-Cl | 90 |
| 27 | $2\text{-}CH_2OH$ | 3-Cl | 75 |
| 28 | 2-CN | $4\text{-}NO_2$ | 209 |

Analog zu den beschriebenen Methoden lassen sich auch die folgenden heterocyclischen Amine herstellen:

2-Amino-4-phenylthiazol
2-Amino-5-nitrothiazol
2-Amino-3-cyan-4-methyl-5- aminocarbonyl-thiophen
2-Amino-5-nito-benzthiazol.

**Patentansprüche**

1. Verfahren zur Spaltung von Phthalimiden der Formel I

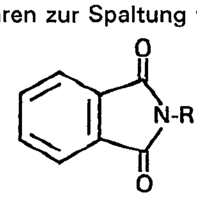

in der

R gegebenenfalls substituiertes Aryl oder Hetaryl ist, dadurch gekennzeichnet, dass man die Verbindungen der Formel I mit Alkanolaminen behandelt.

2. Verfahren gemäss Anspruch 1, dadurch gekennzeichnet, dass man als Alkanolamin Monoethanolamin verwendet.

## Claims

1. A process for the cleavage of a phthalimide of the formula I

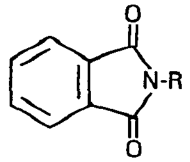

where

R is an optionally substituted aryl or hetaryl, wherein a compound of the formula I is treated with an alkanolamine.

2. A process as claimed in claim 1, wherein the alkanolamine used is monoethanolamine.

## Revendications

1. Procédé pour le clivage de phthalimides de formule I

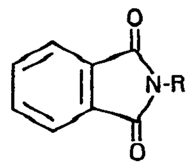

dans laquelle

R est un radical aryle ou hétaryle éventuellement substitué, caractérisé en ce qu'on traite les composés de formule I par des alcanolamines.

2. Procédé selon la revendication 1, caractérisé en ce qu'on utilise, en tant qu'alcanolamine, la monoéthanolamine.